# EUROPÄISCHE PATENTSCHRIFT

(11) **EP 0 662 143 B1**
(45) Veröffentlichungstag und Bekanntmachung des Hinweises auf die Patenterteilung: **19.06.1996**
(21) Anmeldenummer: 93920811.2
(22) Anmeldetag: 23.09.1993
(51) Int. Cl.: C12P 13/22, C12N 15/01, C12N 15/52, C12N 1/20

(54) **MIKROORGANISMEN FÜR DIE PRODUKTION VON TRYPTOPHAN UND VERFAHREN ZU IHRER HERSTELLUNG**
MICROORGANISMS FOR THE PRODUCTION OF TRYPTOPHAN AND PROCESS FOR PRODUCING THE SAME
MICRO-ORGANISMES PRODUCTEURS DE TRYPTOPHANE ET LEUR PROCEDE DE PRODUCTION

(30) Priorität: 28.09.1992 DE 4232468
(43) Veröffentlichungstag der Anmeldung: 12.07.1995
(73) Patentinhaber: Consortium für elektrochemische Industrie GmbH, D-81379 München (DE)
(72) Erfinder: WICH, Günter, D-81543 München (DE); LEINFELDER, Walfred, D-81549 München (DE); BACKMAN, Keith, Bedford, MA 01730 (US)
(74) Vertreter: Potten, Holger
(86) Internationale Anmeldenummer: EP9302588
(87) Internationale Veröffentlichungsnummer: WO9408031

(56) Entgegenhaltungen:
- EP-A- 0 488 424
- WO-A-87/01130
- US-A- 4 371 614
- JOURNAL OF BACTERIOLOGY Bd. 106, Nr. 3 , Juni 1971 Seiten 972 - 982 TETSUYA TOSA AND LEWIS I. PIZER 'Biochemical bases for the antimetabolite action of L-serine hydroxamate' in der Anmeldung erwähnt
- JOURNAL OF BIOLOGICAL CHEMISTRY. (MICROFILMS) Bd. 261, Nr. 26 , September 1986 , BALTIMORE, MD US Seiten 12179 - 12183 TOBEY, K. L., AND G. A. GRANT 'The nucleotide sequence of the serA gene of Escherichia coli and the amino acid sequence of the encoded protein, D-3-phosphoglycerate dehydrogenase'

### SEQUENZPROTOKOLL

(1) ALLGEMEINE INFORMATION:
   (i) ANMELDER:
      (A) NAME: Consortium fuer elektrochemische Industrie GmbH
      (B) STRASSE: Zielstattstr. 20
      (C) ORT: Muenchen
      (E) LAND: Deutschland
      (F) POSTLEITZAHL: 81379
      (G) TELEPHON: 089-62792686
      (H) TELEFAX: 089-62792795
   (ii) ANMELDETITEL: Mikroorganismen fuer die Produktion von Tryptophan und Verfahren zu ihrer Herstellung
   (iii) ANZAHL DER SEQUENZEN: 14
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) L·NGE: 52 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: C-Terminus
   (vi) URSPRÜNLICHE HERKUNFT:
      (A) ORGANISMUS: Escherichia coli
      (B) STAMM: B
   (vii) UNMITTELBARE HERKUNFT:
      (B) CLON: pGC3
   (xi) SEQUENZBESCHREIBUNG: SEQ ID NO: 1:
(2) INFORMATION ZU SEQ ID NO: 2:
   (i) SEQUENZ CHARAKTERISTIKA:
      (A) LÄNGE: 51 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: C-Terminus
   (vi) URSPRÜNLICHE HERKUNFT:
      (A) ORGANISMUS: Escherichia coli
      (B) STAMM: B
   (vii) UNMITTELBARE HERKUNFT:
      (B) CLON: pGH5
   (xi) SEQUENZBESCHREIBUNG: SEQ ID NO: 2:
   Ala Glu Gln Gly Val Asn Ile Ala Ala Gln Tyr Leu Gln Thr Ser Ala
   1               5                   10                  15
   Gln Met Gly Tyr Val Val Ile Asp Ile Glu Ala Asp Glu Asp Val Ala
               20                  25                  30
   Glu Lys Ala Leu Gln Ala Met Lys Ala Ile Pro Gly Thr Ile Arg Ala
           35                  40                  45
   Arg Leu Leu
       50
(2) INFORMATION ZU SEQ ID NO: 3:
   (i) SEQUENZ CHARAKTERISTIKA:
      (A) L·NGE: 56 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: C-Terminus
   (vi) URSPRÜNLICHE HERKUNFT:
      (A) ORGANISMUS: Escherichia coli
      (B) STAMM: B
   (vii) UNMITTELBARE HERKUNFT:
      (B) CLON: pKB1508
   (xi) SEQUENZBESCHREIBUNG: SEQ ID NO: 3:
(2) INFORMATION ZU SEQ ID NO: 4:
   (i) SEQUENZ CHARAKTERISTIKA:
      (A) LÄNGE: 55 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: C-Terminus
   (vi) URSPRÜNLICHE HERKUNFT:
      (A) ORGANISMUS: Escherichia coli
      (B) STAMM: B
   (vii) UNMITTELBARE HERKUNFT:
      (B) CLON: pGH11
   (xi) SEQUENZBESCHREIBUNG: SEQ ID NO: 4:
   Ala Glu Gln Gly Val Cys Ser Arg Ala Asn Ile Ala Ala Gln Tyr Leu
   1 5 10 15
   Gln Thr Ser Ala Gln Met Gly Tyr Val Val Ile Asp Ile Glu Ala Asp
   20 25 30
   Glu Asp Val Ala Glu Lys Ala Leu Gln Ala Met Lys Ala Ile Pro Gly
   35 40 45
   Thr Ile Arg Ala Arg Leu Leu
   50 55
(2) INFORMATION ZU SEQ ID NO: 5:
   (i) SEQUENZ CHARAKTERISTIKA:
      (A) LÄNGE: 47 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: C-Terminus
   (vi) URSPRÜNLICHE HERKUNFT:
      (A) ORGANISMUS: Escherichia coli
      (B) STAMM: B
   (vii) UNMITTELBARE HERKUNFT:
      (B) CLON: pKB1455
   (xi) SEQUENZBESCHREIBUNG: SEQ ID NO: 5:
   Ala Glu Gln Gly Val Asn Ile Ala Ala Gln Tyr Leu Gln Thr Ser Ala
   1               5                   10                  15
   Gln Met Gly Tyr Val Val Ile Asp Ile Glu Ala Asp Glu Asp Val Ala
               20                  25                  30
   Glu Lys Ala Leu Gln Ala Met Lys Ala Ile Pro Gly Thr Ile Arg
           35                  40                  45
(2) INFORMATION ZU SEQ ID NO: 6:
   (i) SEQUENZ CHARAKTERISTIKA:
      (A) LÄNGE: 32 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: inneres
   (vi) URSPRÜNLICHE HERKUNFT:
      (A) ORGANISMUS: Escherichia coli
      (B) STAMM: YMC9
   (xi) SEQUENZBESCHREIBUNG: SEQ ID NO: 6:
   Asn Pro Thr Ala Leu Phe His Gln Leu Cys Gly Asp Arg Pro Ala Thr
   1               5                   10                  15
   Leu Leu Leu Glu Ser Ala Asp Ile Asp Ser Lys Asp Asp Leu Lys Ser
               20                  25                  30
(2) INFORMATION ZU SEQ ID NO: 7:
   (i) SEQUENZ CHARAKTERISTIKA:
      (A) LÄNGE: 32 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (iii) ANTISENSE: NEIN
   (v) ART DES FRAGMENTS: inneres
   (vi) URSPRÜNLICHE HERKUNFT:
      (A) ORGANISMUS: Escherichia coli
      (B) STAMM: PD103
   (xi) SEQUENZBESCHREIBUNG: SEQ ID NO: 7:
   Asn Pro Thr Ala Leu Phe His Gln Leu Cys Gly Asp Arg Pro Ala Thr
   1               5                   10                  15
   Leu Leu Leu Glu Ser Ala Asp Ile Asp Ser Lys Asp Asp Leu Glu Ser
               20                  25                  30
(2) INFORMATION ZU SEQ ID NO: 8:
   (i) SEQUENZ CHARAKTERISTIKA:
      (A) LÄNGE: 32 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: inneres
   (vi) URSPRÜNLICHE HERKUNFT:
      (A) ORGANISMUS: Escherichia coli
      (B) STAMM: KB862
   (xi) SEQUENZBESCHREIBUNG: SEQ ID NO: 8:
   Asn Ser Thr Ala Leu Phe His Gln Leu Cys Gly Asp Arg Pro Ala Thr
   1               5                   10                  15
   Leu Leu Leu Glu Ser Ala Asp Ile Asp Ser Lys Asp Asp Leu Lys Ser
               20                  25                  30
(2) INFORMATION ZU SEQ ID NO: 9:
   (i) SEQUENZ CHARAKTERISTIKA:
      (A) LÄNGE: 32 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: inneres
   (vi) URSPRÜNLICHE HERKUNFT:
      (A) ORGANISMUS: Escherichia coli
      (B) STAMM: SV163
   (xi) SEQUENZBESCHREIBUNG: SEQ ID NO: 9:
   Asn Pro Thr Ala Leu Phe His Gln Leu Cys Gly Asp Arg Pro Ala Thr
   1               5                   10                  15
   Leu Leu Leu Glu Phe Ala Asp Ile Asp Ser Lys Asp Asp Leu Glu Ser
               20                  25                  30
(2) INFORMATION ZU SEQ ID NO: 10:
   (i) SEQUENZ CHARAKTERISTIKA:
      (A) LÄNGE: 32 Aminosäuren
      (B) ART: Aminosäure
      (C) STRANGFORM: Einzel
      (D) TOPOLOGIE: linear
   (ii) ART DES MOLEKÜLS: Protein
   (iii) HYPOTHETISCH: JA
   (v) ART DES FRAGMENTS: inneres
   (vi) URSPRÜNLICHE HERKUNFT:
      (A) ORGANISMUS: Escherichia coli
      (B) STAMM: SV164
   (xi) SEQUENZBESCHREIBUNG: SEQ ID NO: 10:
   Asn Ser Thr Ala Leu Phe His Gln Leu Cys Gly Asp Arg Pro Ala Thr
   1               5                   10                  15
   Leu Leu Leu Glu Ser Ala Asp Ile Asp Ser Lys Asp Asp Leu Glu Ser
               20                  25                  30
(2) INFORMATION ZU SEQ ID NO: 11:
   (i) SEQUENZ CHARAKTERISTIKA:
      (A) LÄNGE: 32 Basenpaare
      (B) ART: Nukleinsäure
      (C) STRANGFORM: beides
      (D) TOPOLOGIE: linear
   (ii) ART DES MOLEKÜLS: DNS
   (iii) HYPOTHETISCH: NEIN
   (iii) ANTISENSE: NEIN
   (vi) URSPRÜNLICHE HERKUNFT:
      (A) ORGANISMUS: in vitro synthetisiertes DNS Fragment
   (xi) SEQUENZBESCHREIBUNG: SEQ ID NO: 11:
   AGCTTATTAC AGCAGACGGG CGCGAATGGA TC
   32
(2) INFORMATION ZU SEQ ID NO: 12:
   (i) SEQUENZ CHARAKTERISTIKA:
      (A) LÄNGE: 12 Basenpaare
      (B) ART: Nukleinsäure
      (C) STRANGFORM: Doppel
      (D) TOPOLOGIE: linear
   (ii) ART DES MOLEKÜLS: DNS
   (iii) HYPOTHETISCH: NEIN
   (iii) ANTISENSE: NEIN
   (vi) URSPRÜNLICHE HERKUNFT:
      (A) ORGANISMUS: in vitro synthetisiertes DNS Fragment
   (xi) SEQUENZBESCHREIBUNG: SEQ ID NO: 12:
   TGCTCTAGAG CA
   12
(2) INFORMATION ZU SEQ ID NO: 13:
   (i) SEQUENZ CHARAKTERISTIKA:
      (A) LÄNGE: 1233 Basenpaare
      (B) ART: Nukleins,,ure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
      (B) STAMM: B
   (ix) MERKMALE:
      (A) NAME/SCHLÜSSEL: CDS
      (B) LAGE: 1..1233
      (C) ART DER ERMITTLUNG: experimentell
      (D) SONSTIGE ANGABEN: /codon_start= 1
         /EC_number= 1.1.1.95
         /product= "D-3-Phosphoglyceratdehydrogenase"
         /evidence= EXPERIMENTAL
         /gene= "serA"
         */standard_name= "serA"*
         /citation= ([1])
   (x) VERÖFFENTLICHUNGSINFORMATION:
      (A) AUTORS: Tobey, K.L. Grant, G.A.
      (B) TITEL: The nucleotide sequence of the serA gene of Escherichia coli and the amino acid sequence of the encoded protein, d-3-phosphoglycerate dehydrogenase
      (C) ZEITSCHRIFT: J. Biol. Chem.
      (D) BAND: 261
      (F) SEITEN: 12179-12183
      (G) DATUM: 1986
   (xi) SEQUENZBESCHREIBUNG: SEQ ID NO: 13:
(2) INFORMATION ZU SEQ ID NO: 14:
   (i) SEQUENZ CHARAKTERISTIKA:
      (A) LÄNGE: 410 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 14:
   Met Ala Lys Val Ser Leu Glu Lys Asp Lys Ile Lys Phe Leu Leu Val
    1               5                  10                  15
   Glu Gly Val His Gln Lys Ala Leu Glu Ser Leu Arg Ala Ala Gly Tyr
               20                  25                  30
   Thr Asn Ile Glu Phe His Lys Gly Ala Leu Asp Asp Glu Gln Leu Lys
           35                  40                  45
   Glu Ser Ile Arg Asp Ala His Phe Ile Gly Leu Arg Ser Arg Thr His
       50                  55                  60
   Leu Thr Glu Asp Val Ile Asn Ala Ala Glu Lys Leu Val Ala Ile Gly
   65                  70                  75                  80
   Cys Phe Cys Ile Gly Thr Asn Gln Val Asp Leu Asp Ala Ala Ala Lys
   85 90 95
   Arg Gly Ile Pro Val Phe Asn Ala Pro Phe Ser Asn Thr Arg Ser Val
   100 105 110
   Ala Glu Leu Val Ile Gly Glu Leu Leu Leu Leu Leu Arg Gly Val Pro
   115 120 125
   Glu Ala Asn Ala Lys Ala His Arg Gly Val Trp Asn Lys Leu Ala Ala
   130 135 140
   Gly Ser Phe Glu Ala Arg Gly Lys Lys Leu Gly Ile Ile Gly Tyr Gly
   145 150 155 160
   His Ile Gly Thr Gln Leu Gly Ile Leu Ala Glu Ser Leu Gly Met Tyr
   165 170 175
   Val Tyr Phe Tyr Asp Ile Glu Asn Lys Leu Pro Leu Gly Asn Ala Thr
   180 185 190
   Gln Val Gln His Leu Ser Asp Leu Leu Asn Met Ser Asp Val Val Ser
   195 200 205
   Leu His Val Pro Glu Asn Pro Ser Thr Lys Asn Met Met Gly Ala Lys
   210 215 220
   Glu Ile Ser Leu Met Lys Pro Gly Ser Leu Leu Ile Asn Ala Ser Arg
   225 230 235 240
   Gly Thr Val Val Asp Ile Pro Ala Leu Cys Asp Ala Leu Ala Ser Lys
   245 250 255
   His Leu Ala Gly Ala Ala Ile Asp Val Phe Pro Thr Glu Pro Ala Thr
   260 265 270
   Asn Ser Asp Pro Phe Thr Ser Pro Leu Cys Glu Phe Asp Asn Val Leu
   275 280 285
   Leu Thr Pro His Ile Gly Gly Ser Thr Gln Glu Ala Gln Glu Asn Ile
   290 295 300
   Gly Leu Glu Val Ala Gly Lys Leu Ile Lys Tyr Ser Asp Asn Gly Ser
   305 310 315 320
   Thr Leu Ser Ala Val Asn Phe Pro Glu Val Ser Leu Pro Leu His Gly
   325 330 335
   Gly Arg Arg Leu Met His Ile His Glu Asn Arg Pro Gly Val Leu Thr
   340 345 350
   Ala Leu Asn Lys Ile Phe Ala Glu Gln Gly Val Asn Ile Ala Ala Gln
   355 360 365
   Tyr Leu Gln Thr Ser Ala Gln Met Gly Tyr Val Val Ile Asp Ile Glu
       370                 375                 380
   Ala Asp Glu Asp Val Ala Glu Lys Ala Leu Gln Ala Met Lys Ala Ile
   385                 390                 395                 400
   Pro Gly Thr Ile Arg Ala Arg Leu Leu Tyr
                   405                 410

## Beschreibung

Die Erfindung betrifft Mikroorganismen für die Produktion von Tryptophan und Verfahren zu ihrer Herstellung.

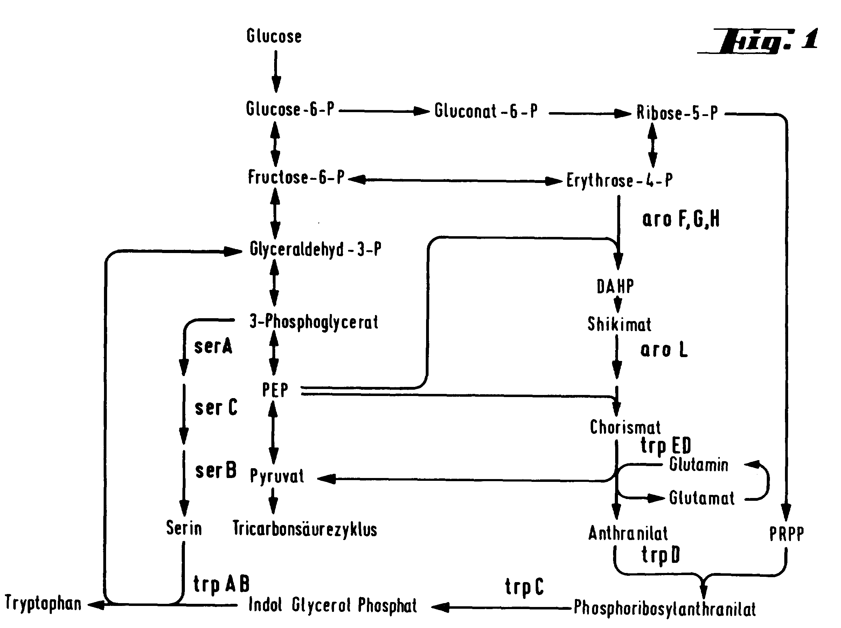

Es ist bekannt, daß der Tryptophanstoffwechsel in allen bisher untersuchten Mikroorganismen über einen einheitlichen Biosyntheseweg verläuft (Somerville, R. L., Herrmann, K. M., 1983, Aminoacids, Biosynthesis and Genetic Regulation, Adelison-Wesley Publishing Company, USA: 301-322 und 351-378; Aida et al., 1986, Biotechnology of amino acid production, progress in industrial microbiology Vol. 24, Elsevier Science Publishers, Amsterdam: 188-206). Der Tryptophanstoffwechsel, seine Verknüpfung mit dem Serinstoffwechsel sowie die für die wichtigsten Enzyme kodierenden Gene sind in Fig. 1 dargestellt.

Bekannte Verfahren zur Tryptophanproduktion beruhen darauf, daß ein mutiertes trpE-Gen, das für eine Tryptophan-insensitive Anthranilatsynthase kodiert, zusammen mit den anderen Genen des trp-Operons auf einem geeigneten autonom replizierbaren Vektor exprimiert wird. Durch die höhere Kopienzahl der Gene kommt es zu einer erhöhten Expression der trp-Gene und entsprechend zu einer erhöhten Menge der einzelnen Enzyme des Tryptophanstoffwechsels. Dies resultiert in einer Überproduktion von Tryptophan.

Beispiele für solche Verfahren sind für eine Reihe von Organismen beschrieben: z.B. für E. coli: EP 0 293 207, US 4,371,614, für Bacillus US 4,588,687, für Corynebakterium und Brevibakterium EP 0 338 474. Bei diesen Verfahren treten eine Reihe von Problemen in der Prozeßführung auf. Es kann zur Instabilität und zum Verlust des Vektors oder zu einem verlangsamten Wachstum des Produktionsstammes kommen.

Die EP-A-0 401 735 (Anmelder: Kyowa Hakko Kogyo Co.) beschreibt ein Verfahren zur Produktion von L-Tryptophan mit Hilfe von Corynebakterien- oder Brevibakterien-Stämmen, die rekombinante Plasmide enthalten. Diese Plasmide tragen die genetische Information zur Synthese der Enzyme DAHP-Synthase, Anthranilatsynthase, Indol-3-Glycerol-P-Synthase, Tryptophansynthase und Phosphoglyceratdehydrogenase. Es werden feedbackresistente Anthranilatsynthase-Allele verwendet.

Es ist weiter bekannt, die Tryptophanproduktion in Stämmen mit dereguliertem Tryptophanstoffwechsel durch das Einbringen von mehreren serA, oder serA, B, C, Wildtyp-Genen zu erhöhen. So beschreiben die Chemical Abstracts CA 111 (1989) 16 86 88q und CA 111 (1989) 16 86 89r die Verwendung von Bacillusstämmen, die das serA-Wildtyp-Allel bzw. alle Wildtyp-Gene des Serinstoffwechsels (serA, serB und serC) auf Plasmiden überexprimieren zur Produktion von Tryptophan. WO-A-87/01130 beschreibt den Einsatz von serA, serB und serC Wildtypallelen zur Produktion von Tryptophan in E. coli.

Die Erhöhung der Tryptophanausbeute durch ein Verhindern des Serinabbaus in der Zelle ist aus EP-A-0 149 539 (Anmelder: Stauffer Chemical Company) bekannt. Diese Patentanmeldung beschreibt E. coli-K12-Mutanten, in denen das Serin abbauende Enzym Serindeaminase (sda) zerstört ist. Sie beschreibt auch den Einsatz solcher Stämme für die Produktion von Aminosäuren. Beispiel VIII beschreibt die Benützung eines solchen Stammes zur Überproduktion von Tryptophan aus Anthranilat. Die im Vergleich zu einem Stamm mit intakter Serindeaminase verbesserte Tryptophanausbeute wird in der europäischen Patentanmeldung damit erklärt, daß in Mikroorganismen, in denen der Vorrat an Tryptophan-Vorstufen sehr hoch ist, Serin bzw. die Serinbiosynthesekapazität ratenlimitierend für die Produktion von Tryptophan ist.

Die Aufgabe der Erfindung war es, Mikroorganismen zur Verfügung zu stellen, die vermehrt Tryptophan produzieren und Verfahren zur Verfügung zu stellen, die die Herstellung solcher Mikroorganismen ermöglichen.

Die Aufgabe wird gelöst durch Stämme von Mikroorganismen, die dadurch gekennzeichnet sind, daß sie einen deregulierten Tryptophanstoffwechsel und einen zumindest durch ein feedbackresistentes serA-Allel deregulierten Serinstoffwechsel besitzen.

Im Sinne der vorliegenden Erfindung sind unter feedbackresistenten serA-Allelen Mutanten des serA-Gens zu verstehen, die für eine Phosphoglyceratdehydrogenase mit einer im Vergleich zur entsprechenden Wildtyp-Phosphoglyceratdehydrogenase des jeweiligen Mikroorganismus verringerten Serin-Sensitivität kodieren.

Durch die erfindungsgemäße Kombination mindestens eines feedbackresistenten serA-Allels mit einem Mikroorganismus mit dereguliertem Tryptophanstoffwechsel erhöhte sich die Tryptophanausbeute erstaunlicherweise um das bis zum 2,6-fache im Vergleich zu der Ausbeute, die mit dem gleichen Mikroorganismus ohne das feedbackresistente serA-Allel bei ansonsten gleichen Kulturbedingungen erzielt werden kann.

Daß die erfindungsgemäßen Stämme vermehrt Tryptophan produzieren, ist unerwartet und überraschend, da feedbackresistente serA-Allele nur bei einem hohen intrazellulären Serinspiegel einen Effekt zeigen (Tosa T, Pizer L.J., 1971, Journal of Bacteriology Vol. 106: 972-982; Winicov J., Pizer L.J., 1974, Journal of Biological Chemistry Vol. 249: 1348-1355). Gemäß dem Stand der Technik (z. B. EP-A-O 149 539) haben Mikroorganismen mit dereguliertem Tryptophanstoffwechsel jedoch einen niedrigen Serinspiegel. Daher ist durch das erfindungsgemäße Einbringen eines feedbackresistenten serA-Allels in Mikroorganismen mit dereguliertem Tryptophan-stoffwechsel keine Erhöhung der Tryptophanproduktion zu erwarten.

Da bei allen bekannten Mikroorganismen der Tryptophanstoffwechsel über den in Fig. 1 dargestellten Stoffwechselweg verläuft und die zur Herstellung der erfindungsgemäßen Stämme anzuwendenden Techniken im Prinzip bekannt und auf alle Mikroorganismen anwendbar sind, sind erfindungsgemäße Stämme aus beliebigen Mikroorganismen herstellbar.

Bevorzugt geeignet zur Herstellung eines erfindungsgemäßen Stammes sind Bakterien. Besonders bevorzugt geeignet sind gramnegative Bakterien, insbesondere E. coli.

Erfindungsgemäße Stämme können dadurch erhalten werden, daß in einem beliebigen Tryptophan-prototrophen Ausgangsstamm die Regulation des Tryptophanstoffwechsels ganz oder teilweise aufgehoben wird und in diesen Stamm ein feedbackresistentes serA-Allel eingebracht wird.

Erfindungsgemäße Stämme können ebenfalls erhalten werden, indem man bei Tryptophan-auxotrophen Ausgangsstämmen die Fähigkeit, Tryptophan zu synthetisieren, wiederherstellt, wobei der rekonstruierte Tryptophanstoffwechsel dereguliert ist, und in solche Stämme ein feedbackresistentes serA-Allel einbringt.

Die Deregulation des Tryptophanstoffwechsels in Mikroorganismen ist durch eine Reihe verschiedener Verfahren, die aus dem Stand der Technik bekannt sind, möglich.

Eine Möglichkeit, den Tryptophanstoffwechsel zu deregulieren ist es, das Enzym Anthranilatsynthase zu verändern. Dieses Enzym katalysiert bei allen Mikroorganismen den ersten Schritt des tryptophanspezifischen Biosynthesewegs. Es wird in seiner Aktivität von Tryptophan gehemmt und reguliert damit, abhängig von der Tryptophanmenge, den Metabolitenstrom durch den Tryptophan-Biosyntheseweg. Das Enzym wird durch das Gen trpE kodiert.

Mutierte trpE-Gene, die für Anthranilatsynthasen mit in Vergleich zur entsprechenden Wildtyp Anthranilatsynthase verminderter Sensitivität gegen Tryptophan kodieren, im folgenden auch als feedbackresistente trpE-Allele bezeichnet, können durch Mutagenese und anschließende Selektion eines Tryptophan-prototrophen Ausgangsstamms erhalten werden. Dazu wird der betreffende Stamm einer Mutationen induzierenden Behandlung unterworfen (Miller J.H., 1972, Experiments in Molecular Genetics, Cold Spring Harbor Laboratory, USA: 113-185).

Der behandelte Stamm wird auf einem Nährmedium angezogen, das mindestens einen Tryptophanantagonisten in einer Menge enthält, die ausreicht, den Stamm im Wachstum zu hemmen. Beispiele für geeignete Tryptophanantagonisten sind 4-Methyltryptophan, 5-Methyltryptophan, 6-Methyltryptophan, halogenierte Tryptophane, Tryptazan, Indol, Indolacrylsäure.

Resistente Klone werden auf die Tryptophan-Sensitivität ihrer Anthranilatsynthase geprüft. Für die Bestimmung der Tryptophan-Sensitivität der Anthranilatsynthase kann jede Methode benützt werden, die es erlaubt, die Aktivität dieses Enzyms in Anwesenheit von Tryptophan zu bestimmen. Beispielsweise kann Chorismat in einem geeigneten Puffersystem mit seinem Reaktionspartner Glutamin unter Enzymkatalyse zur Reaktion gebracht werden (Bauerle R. et al., 1987, Methods in Enzymology Vol. 142: 366-386). Dem Testansatz wurden kinetisch Aliquots entnommen und die pro Zeit entstandene Menge des Reaktionsproduktes Anthranilat wurde durch HPLC-Analyse bestimmt. Die pro Zeit entstandene Menge an Anthranilat ist ein direktes Maß für die Aktivität der Anthranilatsynthase. Der Test wird in An- und Abwesenheit von Tryptophan ausgeführt, um die Sensitivität der getesteten Anthranilatsynthase zu bestimmen.

Es ist ebenso möglich, Tryptophan-insensitive trpE-Allele durch direkte gentechnische Manipulation zu erzeugen. (Bauerle R. et al., 1987, Methods in Enzymology Vol. 142: 366-386). Es sind eine Anzahl von Mutationen in der Aminosäuresequenz der Anthranilatsynthase verschiedener Organismen beschrieben, die zu einer verringerten Sensitivität des Enzyms gegen Tryptophan führen. (Beispielsweise für Salmonella: Caliguiri MG., Bauerle R., 1991, J. of Biol. Chem. Vol. 266: 8328 - 8335; für Brevibakterium, Corynebacterium: Matsui K. et al., 1987, J. Bact. Vol. 169: 5330 - 5332).

Es sind Methoden bekannt, die es ermöglichen in einem DNS-Fragment an spezifischer Stelle eine Mutation einzuführen. Solche Mehoden sind u. a. in folgenden Veröffentlichungen beschrieben:
Sakar G., Sommerauer S. S., 1990, Bio Techniques 8: 404-407, beschreiben polymerase chain reaction abhängige site directed Mutagenese;
Ausubel F. M. et al., 1987, Current Protocols in Molecular Biology, Greene Publishing Associates, beschreiben Phage M13 abhängige Methoden;
Smith M., 1985, Ann. Rev. Genet. 19: 423-462, beschreibt andere Methoden.

Das DNS-Fragment, das das Wildtyp-trpE-Gen umfaßt, wird mit bereits beschriebenen Standardtechniken zur Herstellung rekombinanter DNS auf einen Vektor rekombiniert. Durch Anwendung der vorgenannten Methoden zur site-directed Mutagenese werden ein oder mehrere Nukleotide der DNS-Sequenz so verändert, daß die nun durch das Gen kodierte Aminosäuresequenz der Aminosäuresequenz einer Tryptophan-insensitiven Anthranilatsynthase entspricht. Mit den beschriebenen Techniken lassen sich in ein beliebiges trpE-Gen eine oder mehrere Mutationen einführen, die bewirken, daß die kodierte Anthranilatsynthase eine zur Tryptophan-Insensitivität führende Aminosäuresequenz besitzt.

Zusätzlich sind folgende Eigenschaften im erfindungsgemäßen Stamm wünschenswert aber nicht zwingend notwendig: ein defektes Tryptophanrepressor-Protein, eine defekte Attenuationskontrolle der Expression des trp-Operons, eine defekte Tryptophanase. Diese Eigenschaften lassen sich im erfindungsgemäßen Stamm am einfachsten durch die Auswahl eines Ausgangsstamms, der schon eine oder mehrere der entsprechenden Eigenschaften trägt, erhalten. Die Herstellung oder Auswahl kann durch die Kombination der im folgenden genannten Selektionsmethoden erfolgen.

Das Tryptophanrepressor-Protein ist ein übergeordnetes Regulatorprotein der Tryptophanbiosynthese. Zusammen mit Tryptophan als Aporepressor reprimiert dieses Protein die Expression der trp-Operon-Gene. Das Protein wird durch das Gen trpR kodiert. Tryptophanrepressor-Mutanten können z.B. unter Mutanten, die gegen Tryptophanantagonisten, wie z.B. 5-Methyltryptophan, resistent sind, selektiert werden. Beispiele beschreibt J. Mol. Biol. 44, 1969, 185-193 oder Genetics 52, 1965, 1303-1316.

Neben der Kontrolle durch das trpR-kodierte Protein unterliegt das trp-Operon zusätzlich einer Attenuationskontrolle. Verantwortlich für die Regulation ist eine DNS-Region vor dem ersten Gen des trp-Operons. Mutationen oder Deletionen in diesem Bereich können zu einer Deregulation führen. Solche Mutanten können unter Mutanten, die gegen Tryptophanantagonisten wie 5-Methyltryptophan resistent sind, selektiert werden. Zum anderen können solche Mutationen, besonders aber Deletionen, erhalten werden, indem man durch Verfahren der site-directed-Mutagenese diese Veränderung ortsspezifisch im Attenuationsbereich der DNS induziert. Durch die bereits beschriebenen Techniken der ortsspezifischen Mutagenese ist es möglich, den inaktivierten Attenuatorbereich ins Chromosom des erfindungsgemäßen Stamms an die Stelle des natürlichen Attenuatorbereiches zu rekombinieren.

Das Enzym Tryptophanase (tnaA) katalysiert den Abbau von Tryptophan zü Indol, Pyruvat und NH₃. Es ist wünschenswert, daß dieses Enzym in Tryptophan-Produktionsstämmen inaktiv ist. Stämme, die in diesem Enzym defekt sind, können erhalten werden, indem man die Organismen einer mutagenen Behandlung unterwirft und unter den Mutanten solche sucht, die nicht mehr in der Lage sind, Tryptophan als Kohlenstoff- und Stickstoffquelle zu benützen. Ein detailliertes Beispiel beschreibt J. Bact. 85, 1965, 680-685. Alternativ ist es ebenso möglich, mit oben genannten Techniken ortsspezifisch Deletionen in das tnaA-Gen einzuführen, die zur Inaktivierung führen.

Eine Reihe von zusätzlichen Mutationen des Ausgangsstammes sind geeignet, eine weitere Erhöhung der Tryptophanproduktion zu bewirken. So ist bevorzugt neben dem Tryptophanbiosyntheseweg zusätzlich der allgemeine Biosyntheseweg der aromatischen Aminosäuren (Shikimisäureweg) regulationsinsensitiv. Deshalb sind Stämme, die eine regulationsinsensitive Dehydroarabinoheptulosonsäuresynthase besitzen und deren Tyrosinrepressorprotein (tyrR) durch eine Mutation oder Deletion inaktiviert ist, als Ausgangsstämme für die Herstellung der erfindungsgemäßen Stämme bevorzugt. Ebenso sind Stämme bevorzugt, deren Phenylalanin und Tyrosinstoffwechsel gestört ist. Dadurch wird der ausschließliche Fluß des Vorläufermoleküls Chorismat ins Tryptophan gewährleistet. Solche Stämme haben beispielsweise Mutationen oder Deletionen in den Genen pheA und/oder tyrA.

Eine Reihe von Stämmen sind bekannt, die in einzelnen oder mehreren Schritten der Tryptophanbiosynthese oder dem Shikimisäureweg dereguliert sind und Tryptophan überproduzieren. Beispiele sind Bacillus subtilis FermBP-4, FermP1483 (DE 3123001), Brevibacterium flavum ATCC 21427 (US 3,849,251), Corynebacterium glutamicum, ATCC 21842-21851 (US 3,594,279, US 3,849,251), Micrococcus luteus ATCC 21102 (US 3,385,762), E.coli ATCC 31743 (CA 1182409). Diese Stämme sind ebenfalls als Ausgangsstämme zur Herstellung der erfindungsgemäßen Stämme geeignet. Sie zeigen, daß Tryptophanproduktionsstämme gemäß der Erfindung in verschiedensten Organismengruppen erhalten werden können.

Neben den Stämmen mit dereguliertem Tryptophanstoffwechsel wird zur Herstellung der erfindungsgemäßen Stämme mindestens ein Gen, welches für eine Phosphoglyceratdehydrogenase mit im Vergleich zur entsprechenden Wildtyp-Phosphoglyceratdehydrogenase verringerter Serin-Sensitivität kodiert, benötigt.

Die Phosphoglyceratdehydrogenase (PGD) wird durch das Gen serA kodiert. Die Sequenz des Wildtyp-serA-Gens ist bekannt. (Tobey K.L., Grant G.A., 1986, J. Bac. Vol. 261, No. 26: 1279-1283). Die Überexpression des Wildtyp-serA-Genprodukts über einen Plasmid-Vektor ist ebenfalls bekannt (Schuller et al., 1989, J. Biol. Chem. Vol. 264: 2645-2648).

Die Herstellung von feedbackresiszenten serA-Allelen mit klassischen genetischen Verfahren wird bei Tosa T., Pizer L.T., 1971, J. Bac. Vol. 106, No. 3: 972-982, beschrieben. Die Selektion erfolgte dabei über die Resistenz der Mutanten gegen das Serinanalogon Serinhydroxamat. Die Mutationen wurden in dieser Literaturstelle nicht näher charakterisiert; der Einfluß der Mutation auf den Stoffwechsel wurde nicht untersucht.

Feedbackresistente serA-Allele sind beispielsweise auch dadurch erhältlich, daß man einen Mikroorganismus einer Mutagenese unterwirft. Als Mutagen kommen UV-Licht und jegliche chemischen Mutagene in Frage, wie z.B. Ethylmethansulfonat oder N-Methyl-N'nitro-N-nitrosoguanidin. Dosierung und Expositionsdauer des gewählten Mutagens werden nach herkömmlichem Verfahren bestimmt (Miller J.H., 1972, Experiments in Molecular Genetics, Cold Spring Harbor Laboratory, USA: 113-143).

Mutagen-behandelte Organismenpopulationen werden einer Selektion auf Klone mit serA-Genen, die für Serin-insensitive Phosphoglyceratdehydrogenasen kodieren, unterworfen. Beispielsweise wird eine mutagen-behandelte Population auf festem Wachstumsmedium, das Serinhydroxamat in ausreichender Menge enthält, um das Wachstum nichtresistenter Bakterien zu hemmen, inkubiert. Resistente Klone werden auf die Serin-Sensitivität ihrer Phosphoglyceratdehydrogenase getestet. Eine beispielhafte Ausführungsform dieses Verfahrens ist bei Tosa and Pfizer, 1971, J. Bact. 100, 3: 972-982, beschrieben.

Allele, die für eine Serin-insensitive Phosphoglyceratdehydrogenase kodieren, können ebenfalls durch "genetic engineering" Techniken erzeugt werden.

Die Region der PGD, die die Serin-Regulation vermittelt, liegt im C-terminalen Bereich des Proteins. Es werden daher Insertionen, Substitutionen oder Deletionen von einzelnen oder mehreren Aminosäuren, bevorzugt in den C-terminalen 25 % des PGD-Proteins, besonders bevorzugt in den 50 C-terminalen Aminosäuren des PGD-Proteins, eingeführt. Diese führen zu einer verminderten Sensitivität der PGD gegen Serin.

Allele, die für solche PGDs kodieren, werden dadurch erhalten, daß der 3'-Bereich des serA-Gens, der für die genannten C-terminalen Bereiche der PGD kodiert, verändert wird. Dazu wird das unmutierte serA-Gen durch Benützen dem Fachmann bekannter Techniken zur Herstellung rekombinanter DNS, wie Restriktion, Ligation und Transformation (Maniatis T., Fritsch E.F. und Sambrook J., Molecular Cloning: A Laboratory Manual 1982, Cold Spring Harbor Laboratory), auf einen Klonierungsvektor rekombiniert. Spezifische Veränderungen im 3'-Bereich des Strukturgens können beispielsweise über Techniken der site-directed-Mutagenese erreicht werden.

Beispiele von Serin-insensitiven PGDs, die zur Expression in Mikroorganismen mit dereguliertem Tryptophanstoffwechsel geeignet sind, sind durch Darstellung ihrer C-terminalen Aminosäuresequenz in Tabelle 1 aufgeführt. Bis auf den dargestellten Bereich unterscheiden sich die Proteinsequenzen der Enzyme nicht von der Wildtypsequenz.

Um die Genprodukte der serA-Allele auf PGD-Aktivität und Serin-Sensitivität zu prüfen, wurden folgende Tests benutzt:

Die PGD-Aktivität wurde durch Nachweis der Hin- oder Rückreaktion des Enzyms nach der Methode von Mc Kitrick, J.C. and Lewis J.P., 1980, J. Bact. 141: 235-245, bestimmt. Die Aktivität des Enzyms wird dabei ohne Serin und mit verschiedenen Konzentrationen an Serin gemessen. Der genannte Test ist zur Bestimmung der Serin-Sensitivität jeder Phosphoglyceratdehydrogenase geeignet. Jede andere Methode zur Messung der PGD-Aktivität kann ebenfalls eingesetzt werden.

Als Maß für die Serin-Sensitivität des Enzyms dient der Ki-Wert, d.h. die Serinkonzentrationen, die die Aktivität des Enzyms zu 50 % hemmen. Die Kᵢ-Werte und C-terminalen Aminosäuresequenzen mehrerer feedback-resistenter serA-Allele sowie des Wildtyp-serA-Gens (serAWT) sind in Tabelle 1 aufgeführt.

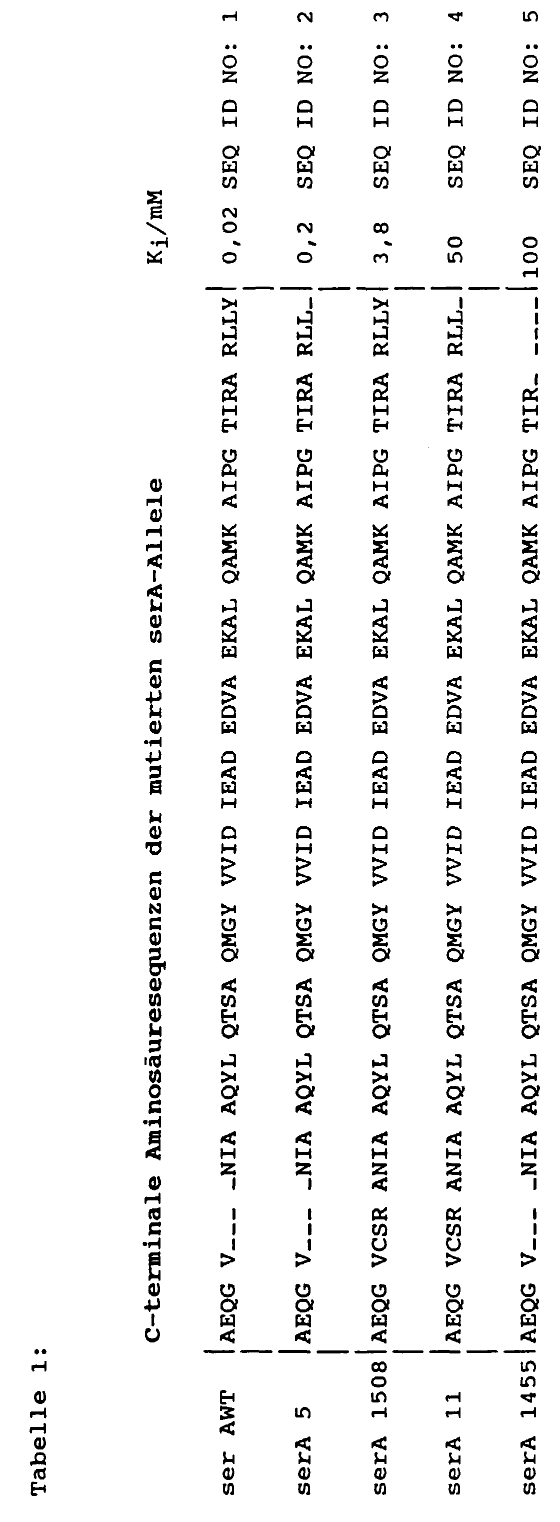

Für die Herstellung der erfindungsgemäßen Stämme bevorzugt geeignet sind überraschenderweise serA-Allele mit einem Ki-Wert zwischen 100 µM und 50 mM Serin.

Zur Expression der PGD-Proteine im erfindungsgemäßen Stamm kann jeder rekombinante Vektor, der zur Expression der Serin-insensitiven serA-Allele führt, eingesetzt werden. Ein zur Herstellung der erfindungsgemäßen Stämme geeigneter rekombinanter Vektor umfaßt mindestens ein serA-Gen, das für eine PGD kodiert, die eine gegenüber dem Wildtyp erniedrigte Sensitivität gegen Serin aufweist und einen Vektoranteil, der im Empfängerstamm autonom replizierbar ist.

Beispiele für Vektoren, die in E. coli autonom replizierbar sind, sind bei Pouwels P.H., Enger-Valk B.e., Brammar W.J. 1985, Cloning Vectors, Elsevier, Amsterdam aufgeführt. Solche Vektoren sind unter anderem:
- Plasmide mit hoher Kopienzahl wie z.B. pBR322; pUC12,
- Plasmide mit mittlerer Kopienzahl wie z.B. pACYC184,177,
- Plasmide mit niedriger Kopienzahl wie z.B. pSC101,
- Phagenvektoren wie z.B. M13, λ-Vektoren.

Vergleichbare Vektoren sind für eine große Anzahl von Bakterien beschrieben (z.B. EP 0 401 735 für Corynebakterien und Brevibakterien oder in CA 111 (1989) 168688q).

Bevorzugt geeignet sind Vektoren mit mittlerer bis niedriger Kopienzahl; besonders bevorzugt sind Vektoren mit einem p15A-Replikon, wie pACYC184 (ATCC37033) oder pACYC177 (ATCC37031).

Eine große Anzahl von Vektoren für andere Bakterien sind in der Literatur beschrieben. (Pouwels et al., 1985, Cloning Vectors, Elsevier Science Publishers, Amsterdam).

Geeignete rekombinante Vektoren können mit den Standardtechniken zur Herstellung rekombinanter DNS erzeugt werden. Diese Techniken sind ausführlich zum Beispiel in Maniatis T., Fritsch E.F. und Sambrook J., 1982, Molecular Cloning: A Laboratory Manual, Cold Spring Harbor Laboratory, USA oder Ausubel F.M. et al, 1987, Current Protocols in Molecular Biology, Greene Publishing Associates, USA, dargestellt.

Die Herstellung rekombinanter Vektoren ist zum Beispiel dadurch möglich, daß die DNS eines Spenderorganismus, der ein serA-Allel im Chromosom oder auf einem rekombinanten Vektor besitzt, das für eine gegen Serin-insensitive PGD kodiert, mit Restriktionsenzymen fragmentiert wird. Die fragmentierte DNS wird mittels konventioneller Methoden, z.B. durch den Einsatz des Enzyms T4-DNA-Ligase, in ein ebenfalls durch Restriktionsenzyme linearisiertes Vektormolekül ligiert. Das Ligationsgemisch wird benützt, um Empfängerstämme mit dereguliertem Tryptophan-Stoffwechsel nach bekannten Verfahren, wie Calciumchloridschock oder Elektroporation, zu transformieren. Vektoren, die die gewünschten serA-Allele enthalten, können in Empfängerstämmen beispielsweise durch oben genannte Verfahren, wie Selektion auf Antibiotika-Resistenz oder Komplementierung von serA-Mutationen, erhalten werden.

In einer weiteren Ausführungsform der erfindungsgemäßen Stämme sind die serA-Allele als Einzelkopie ins Chromosom integriert. Dies kann zum einen dadurch erreicht werden, daß oben beschriebene Mutagenese- und Selektionsstrategien direkt mit einem tryptophanderegulierten Ausgangs stamm durchgeführt werden. Es ist zum anderen auch möglich, serA-Allele, die auf rekombinanten Vektoren vorliegen, ins Chromosom des Produktionsstamms zu integrieren. Eine Anzahl von Methoden zu einer solchen Integration sind bekannt. Beschreibungen für diese Techniken finden sich in folgenden Veröffentlichungen:
Phage Lambda vermittelte Integration: Balakrishnan and Backmann, 1988, Gene 67: 97-103; Simons R.W. et al., 1987, Gene 53: 85-89;
recD abhängiges Genreplacement: Shervell et al., 1987, J. Bact. 141: 235-245;
Weitere Methoden: Silhavy et al., 1988, Experiments with Gene Fusions, Cold Spring Harbor Laboratory.

Bei der Fermentation von erfindungsgemäßen Stämmen zeigte sich völlig überraschend, daß Stämme, enthaltend ein feedbackresistentes serA-Allel mit einem Kᵢ-Wert für Serin zwischen 100 µM und 50 mM und einen deregulierten Tryptophanstoffwechsel, enthaltend ein trpE-Allel mit einem Ki-Wert für Tryptophan zwischen 0,1 mM und 20 mM, die höchste Ausbeute an Tryptophan liefern.

Die folgenden Beispiele dienen der weiteren Erläuterung der Erfindung.

### Beispiel 1:

### Screening auf feedbackresistente trpE-Allele und Integration dieser Allele in das Chromosom

Für die Suche nach feedbackresistenten trpE-Allelen wurde das Tryptophan-Analogon 5-Methyltryptophan eingesetzt. Als mutagenes Agens diente N-Methyl-N'-nitro-N-nitroso-guanidin (NG). Der verwendete Ausgangsstamm war E.coli K12 YMC9 ATCC33927. Die Vorgehensweise bei der Mutagenese richtete sich nach den Angaben von Miller (Miller J. H., 1972, Experiments in Molecular Genetics. Cold Spring Harbor Laboratory, Cold Spring Harbor, N.Y.: 125-129).

Ca. 2 x 10⁹ Zellen von YMC9 aus der exponentiellen Wachstumsphase einer in LB gewachsenen Kultur wurden in 4 ml 0.1 M NaCitrat-Puffer pH 5.5 für 30 min. bei 37°C mit 50 µg/ml NG inkubiert. Nach zweimaligem Waschen mit 0.1 M Phosphatpuffer pH 7.0 wurden 0.1 ml Zellen über Nacht bei 37°C unter Schütteln in LB bebrütet. Anschließend wurden 0.1 ml Zellen aus verschiedenen Verdünnungen (10⁻³, 10⁻⁴, 10⁻⁵ in 0,9 % NaCl) auf Minimalmedienplatten mit 100 µg/ml 5-Methyltryptophan ausgebracht. Neben 5-Methyltryptophan enthielt das Minimalmedium 5 g/l Glukose, 5 mg/l Vitamin B1, 3 g/l KH₂PO₄, 12 g/l K₂HPO₄, 0.3 g/l MgSO₄ x 7 H₂O, 0.1 g/l NaCl, 5 g/l (NH₄)₂SO₄, 14.7 mg/l CaCl₂ x 2H₂O, 2 mg/l FeSO₄ x 7H₂O, 1 g/l Na₃-Citrat und 15 g/l Agar. Nach 24-48 h bei 37°C wurden 5-Methyltryptophan-resistente Klone abgeimpft und auf obigen Platten vereinzelt.

Zur Charakterisierung der erhaltenen Mutanten wurde der Ki-Wert des trpE-Genproduktes für Tryptophan bestimmt (Bauerle R. et al., 1987, Methods in Enzymology Vol. 142: 366-386). Die Mutanten konnten dabei in zwei Klassen eingeteilt werden. Mutanten der Klasse 1 besaßen feedbackresistente Anthranilatsynthasen. Mutanten der Klasse 2 besaßen Enzyme mit erhöhter Anthranilatsynthase-Aktivität bei nicht verändertem Kᵢ-Wert.

Zur Charakterisierung auf molekularer Ebene wurden die relevanten DNA-Bereiche der verschiedenen Mutanten kloniert und sequenziert. Zu diesem Zweck wurde die jeweilige chromosomale DNA isoliert und in einem Ansatz mit den Restriktionsenzymen NheI und ClaI gespalten. Fragmente einer Größe von ca. 5 kb wurden isoliert und mit dem 4158 bp großen NheI/ClaI-Fragment von pBR322 ligiert. Der Ligationsansatz wurde in einen trpE-Stamm von E.coli KB 862 (DSM 7196) transformiert. Selektiert wurde auf Klone, die auf Minimalmedium ohne Tryptophan wachsen konnten. Die komplementierenden Plasmide enthielten alle ein 5 kb NheI/ClaI-Fragment. Neben den Genen trpE und trpD enthält dieses 5kb NheI/ClaI-Fragment auch DNA-Bereiche stromaufwärts von trpE (ca. 0.8 kb) und stromabwärts von trpD (ca. 1kb).In Tabelle 2 sind die Aminosäuresequenzunterschiede und die Kᵢ-Werte von 4 Mutanten der Klasse 1 aufgelistet. In den nicht dargestellten Bereichen stimmen die Sequenzen der Mutanten mit der Wildtyp-Sequenz überein.

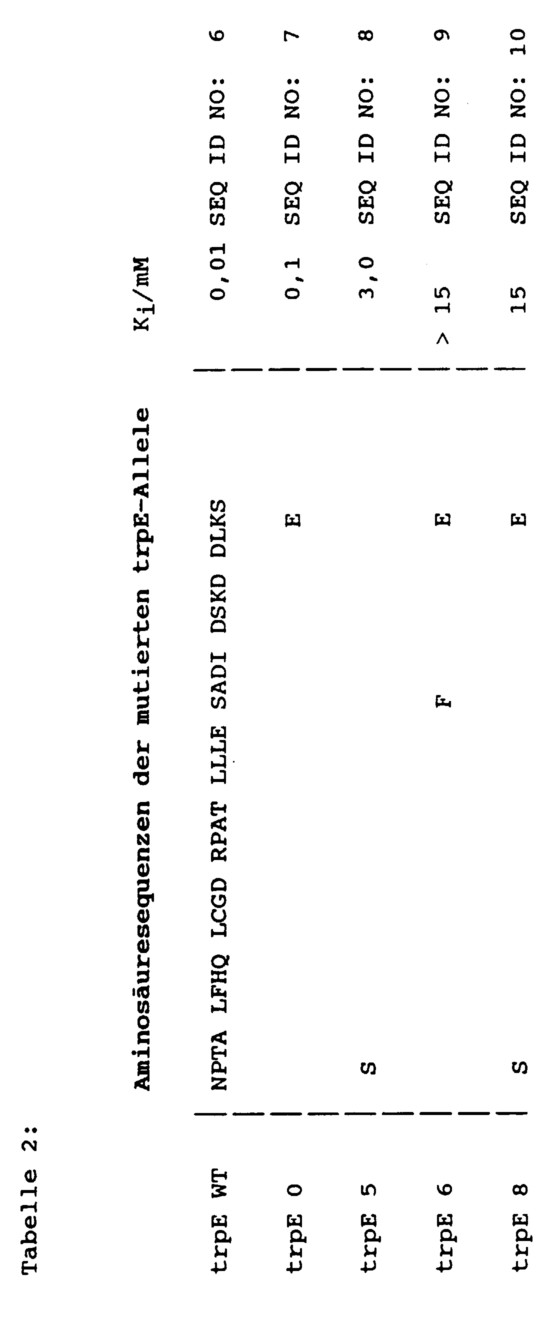

Die Sequenzanalyse der Mutanten der Klasse 2 zeigte, daß Mutationen entweder im Operator-Bereich des trp-Promotors oder im DNA-Bereich, der für das trp-Leaderpeptid kodiert, vorlagen. Die mit ΔtrpL1 bzw. ΔtrpL2 bezeichneten Mutationen haben eine 136 bp bzw. 110 bp große Deletion im DNA-Bereich, der für das Leaderpeptid kodiert. Bei der ΔtrpL1 Mutation umfaßt die Deletion den Bereich von Nukleotidposition 33 bis zur Position 168, bei der ΔtrpL2 Mutation den Bereich von Nukleotidposition 11 bis 120 in der unter der AC-Nummer VOO372 in der EMBL-Datenbank gespeicherten Sequenz.

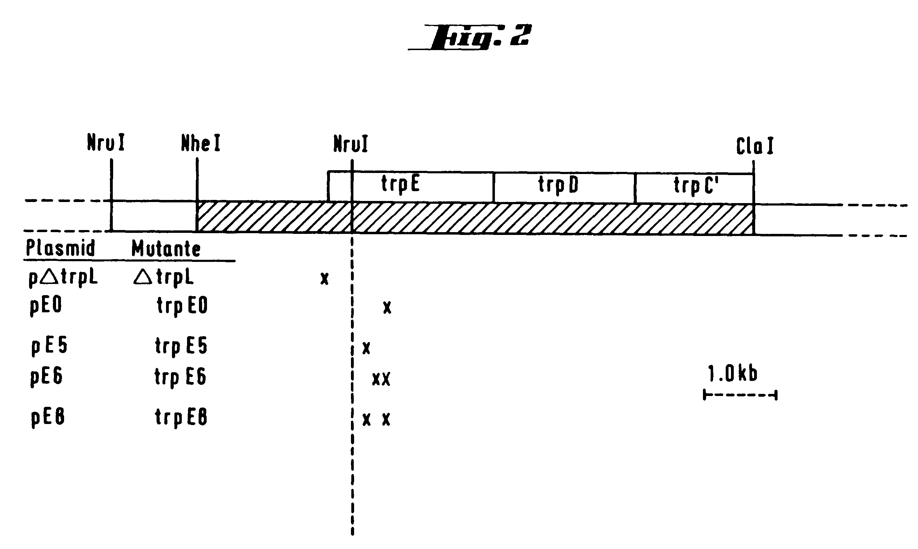

Um eine stärkere Expression der feedbackresistenten trpE-Allele zu erreichen, wurden beide Mutantenklassen kombiniert. Dazu wurde die Mutation ΔtrpL1 der Klasse 2 verwendet. Fig. 2 zeigt schematisch die Lage der ΔtrpL1-Mutation (Klasse 2) und der Mutationen trpE0, trpE5, trpE6 und trpE8 (Klasse 1).

Aus dem Plasmid pΔtrpL (Fig. 2) wurde das 1.6 kb NruI-Fragment, welches die ΔtrpL1-Mutation trägt, isoliert und gegen das entsprechende Wildtyp-NruI-Fragment der Plasmide pEO, pE5, pE6 bzw. pE8 (Fig. 2) ausgetauscht. Die erhaltenen Plasmide wurden mit pIE0, pIE5, pIE6 bzw. pIE8 benannt und für die chromosomale Integration durch homologe Rekombination verwendet. Dazu wurde aus den genannten Plasmiden das jeweilige, ca. 5 kb große chromosomale NheI/ClaI-Fragment wie in Beispiel 2 beschrieben aus low melting Agarose isoliert und in linearer Form in den recD-Stamm PD106 [ΔtrpLD102] eintransformiert. Als Transformationsmethode wurde die CaCl₂-Methode nach Cohen et al., 1972, Proc. Natl. Acad. Sci. USA 69: 2110-2114, benutzt. Der Stamm PD106 wurde gemäß dem Budapester Vertrag am 28.07.1992 bei der Deutschen Sammlung für Mikroorganismen (DSM) unter der Nummer 7195 (DSM 7195) hinterlegt. Selektiert wurde auf Klone, die auf Minimalmedium ohne Tryptophan wachsen konnten und Ampicillin-sensitiv, d.h. Plasmid-frei, waren. Durch P1-Transduktion (Miller J.H., 1972, Experiments in Molecular Genetics. Cold Spring Harbor, N.Y.: 201-205) wurden die trpE-Allele, die für unterschiedlich feedback-resistente trpE-Enzyme kodieren und jeweils mit der ΔtrpL1-Mutation kombiniert sind, aus den jeweiligen Stämmen in KB862 überführt. Der Stamm KB862 wurde gemäß dem Budapester Vertrag am 28.07.1992 bei der Deutschen Sammlung für Mikroorganismen (DSM) unter der Nummer 7196 (DSM 7196) hinterlegt. Die Selektion erfolgte auf Wachstum auf Tryptophan-freiem Minimalmedium. Die erhaltenen Stämme wurden mit PD103(trpE0), KB862(trpE5), SV164 (trpE8) und SV163(trpE6) bezeichnet.

### Beispiel 2:

### Herstellung von serA-Genen, die für Serin insensitive Phosphoglyceratdehydrogenasen kodieren.

Das serA-Wildtyp-Gen wurde aus dem E. coli Stamm E. coli B (ATCC 23226) auf den Plasmidvektor pUC18 kloniert.

Um die chromosomale DNS dieses Stammes zu gewinnen, wurde er über Nacht bei 37°C in Luria-Broth angezogen. Die Bakterienzellen wurden durch Zentrifugation (4000 g) geerntet. Die Lyse der Zellen und die Reinigung der DNS erfolgte nach dem in Ausubel et al., 1987, 2.4.1 - 2.4.2, Current Protocols in Molecular Biology, Greene Publishing Associates, beschriebenen Protokoll. Die erhaltene DNS-Menge wurde spektrophotometrisch bei einer Wellenlänge von 260 nm bestimmt. Die Ausbeuten lagen bei 600 µg/100 ml.

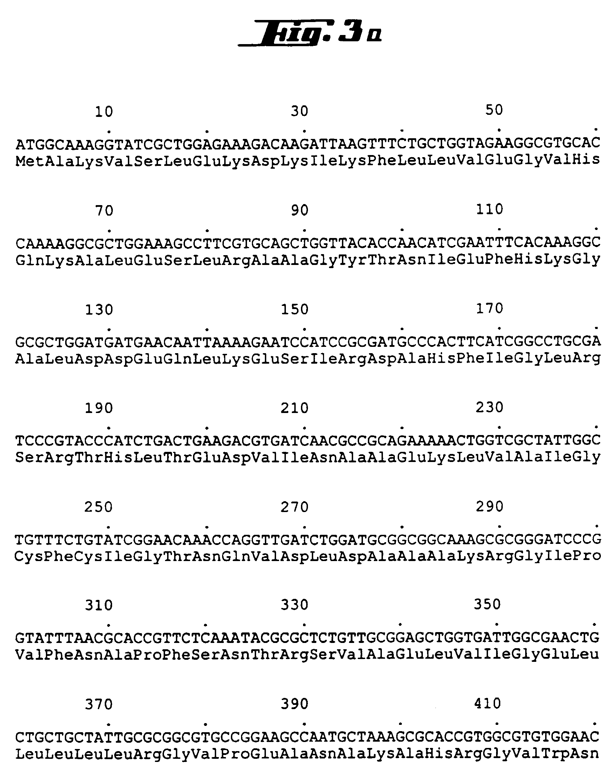

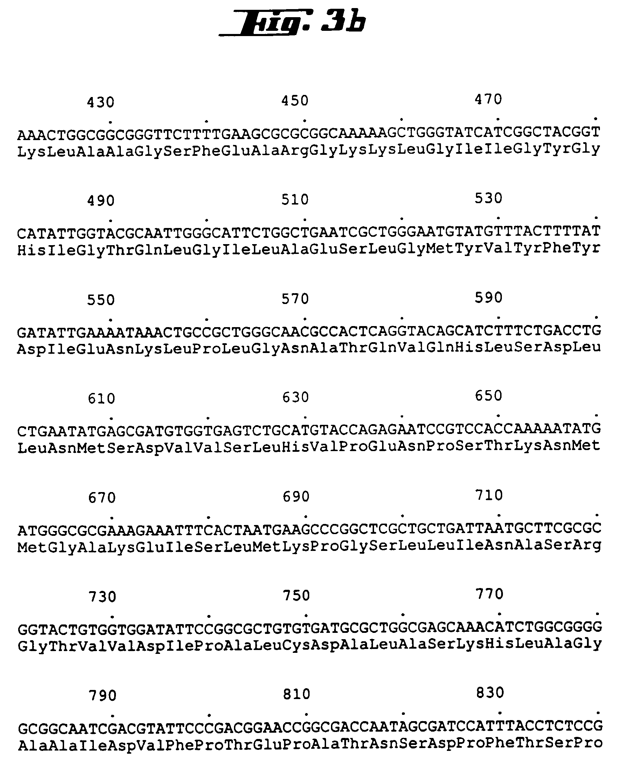

10 µg der chromosomalen DNS wurden mit dem Restriktionsenzym SphI (Boehringer Mannheim GmbH) unter den vom Hersteller angegebenen Bedingungen gespalten. Etwa 3 µg des Fragmentgemisches wurde mit 0.2 µg des ebenfalls SphI-geschnittenen autonom replizierbaren Plasmidvektors pUC18 (Fa. Boehringer Mannheim GmbH) durch das Enzym T₄-Ligase (Fa. Boehringer Mannheim GmbH) unter den vom Hersteller vorgeschriebenen Bedingungen ligiert. Das Ligationsgemisch wurde benützt, um die serA-Mutante PC1523 (CGSC#:5411;) (CGSC: E.coli Genetic Stock Center, Department of Biology 255 OML, Yale University, Postbox 6666, New Haven, CT, USA) zu transformieren. Als Transformationsmethode wurde die CaCl-Methode von Cohen et al., 1972, Proc. Natl. Acad. Sci USA 69: 2110-2114, verwendet. Die transformierten Bakterien wurden auf Minimalmedium ohne Serin ausplattiert. Klone, die ohne Serin wuchsen, enthielten das serA-Gen aus E. coli auf einem 3,5 kb großen SphI-Fragment. Die Sequenz des Wildtyp SerA-Gens ist in Fig. 3 (SEQ ID NO: 13; SEQ ID NO: 14) wiedergegeben. Der rekombinante Vektor mit dem serA-Gen wurde als pGC3 bezeichnet (Fig. 4).

Das serA-Allel serA5 wurde hergestellt, indem das Plasmid pGC3 mit den Restriktionsenzymen SalI und KpnI (Fa.Boehringer Mannheim GmbH) gemäß den Angaben des Herstellers geschnitten wurde. Die erhaltenen Fragmente wurden durch Agarosagelelektrophorese aufgetrennt. Das 2,0 kb große SalI-KpnI-Fragment, das das vollständige serA-Gen bis auf die 8 C-terminalen Kodons enthält, wurde aus dem Gel gereinigt. Dazu wurde die Elektrophorese auf "low-melting"-Agarose (Fa. Boehringer Mannheim GmbH) durchgeführt, so daß die DNS durch einfaches Aufschmelzen der Agarose zurückgewonnen werden konnte. 0,2 µg dieses Fragments wurden mit äquimolaren Mengen eines HindIII/SalI-geschnittenen pUC18 und eines synthetisch hergestellten, doppelsträngigen Oligonukleotides durch T₄-Ligase (Fa. Boehringer Mannheim GmbH) gemäß Herstellerangaben ligiert. Die Nukleotidsequenz dieses Oligonukleotides ist wie folgt:

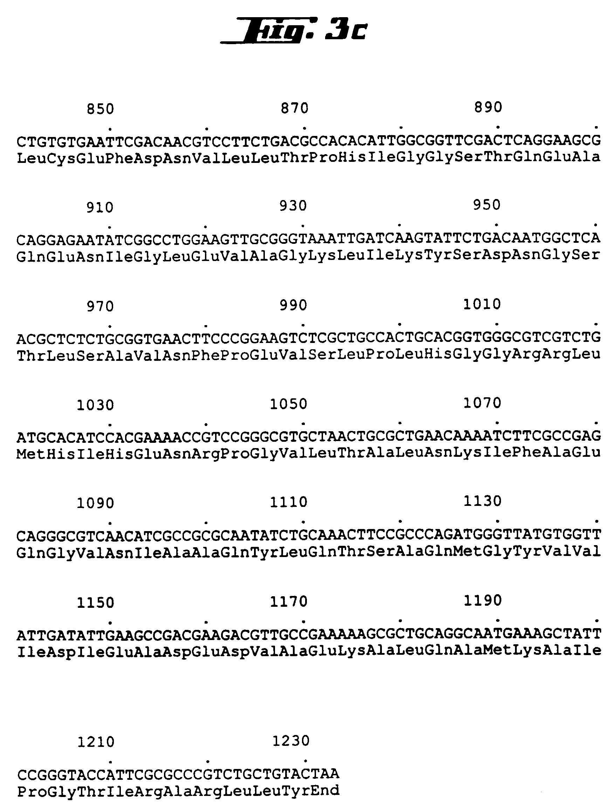

Dieses Oligonukleotid ergänzt 7 der 8 letzten C-terminalen Kodons des serA-Gens. Anstelle des achten Kodons wird das Stoptriplett TAA eingeführt. Die durch dieses serA-Gen kodierte Phosphoglyceratdehydrogenase ist damit um eine Aminosäure C-terminal verkürzt. Die Aminosäuresequenz der mutierten PGD ist in Tabelle 1 (serA5) gezeigt. Das rekombinante Plasmid wurde als pGH5 bezeichnet (Fig. 5). Mit dem Ligationsansatz wurde die serA-Mutante PC1523 transformiert.

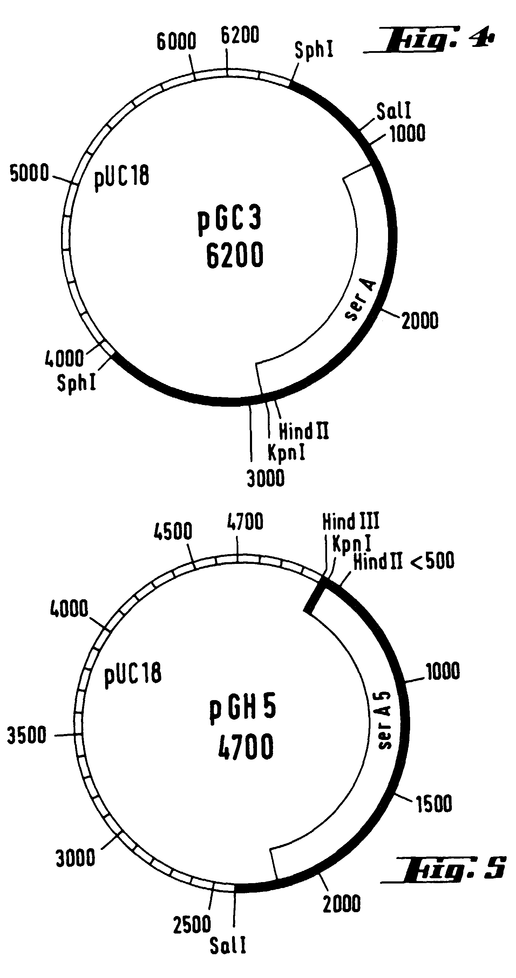

Das serA-Allel serAl508 wurde wie folgt hergestellt. Das Plasmid pGC3 wurde mit SphI/SalI (Fa. Boehringer Mannheim GmbH) gemäß den Angaben des Herstellers geschnitten. Ein 3 kb-Fragment, das das vollständige serA-Gen trägt, wurde gel-elektrophoretisch gereinigt und mit dem SphI/SalI geschnittenen Vektor pUC18 ligiert. Das resultierende Plasmid wurde als pKB1321 bezeichnet (Fig. 6).

pKB1321 wurde mit der Restriktionsendonuklease HindII (Fa. Boehringer Mannheim GmbH) unter Bedingungen inkubiert, die nur ein partielles Schneiden erlauben (0,05 U Enzym pro 1 µg DNS für 10 min., übrige Reaktionsbedingungen gemäß Angaben des Herstellers). Dadurch entsteht unter anderem eine Fraktion von Fragmenten, die an der Position 1793 des serA-Gens HindII geschnitten ist. An dieser Stelle wird durch Ligation ein DNS-Linker mit einer XbaI-Schnittstelle eingefügt. Die Sequenz des DNS-Linkers war wie folgt:

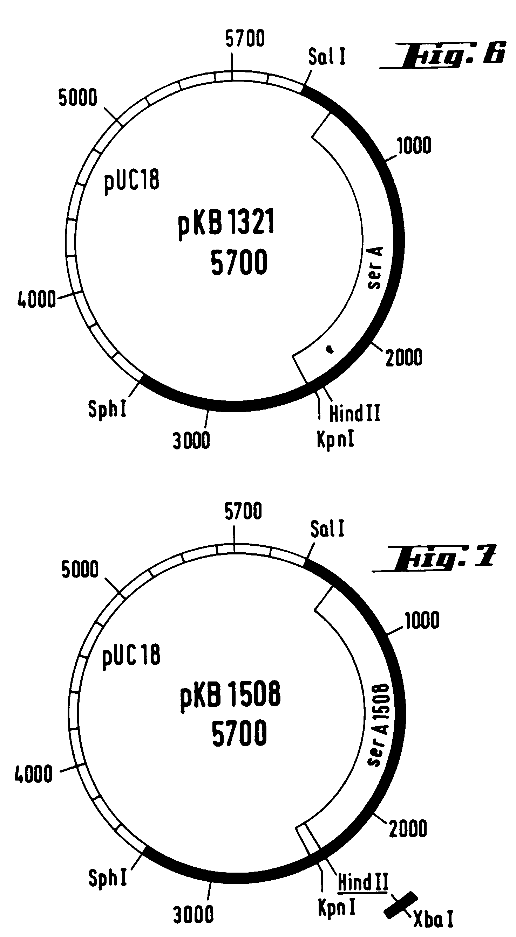

Die Insertion führt zu einer PGD, die an dieser Stelle 4 zusätzliche Aminosäuren trägt. Ihre Sequenz ist in Tabelle 1 dargestellt. Das Plasmid mit der Insertion wurde als pKB 1508 bezeichnet (Fig. 7). Es wurde in die serA-Mutante PC1523 transformiert.

Das serA-Allel serA11 wurde hergestellt, indem das Plasmid pGH5 mit SalI und KpnI (Fa. Boehringer Mannheim GmbH) gemäß den Angaben des Herstellers geschnitten wurde und das 2,8 kb große Fragment des Fragmentgemisches nach Gelelektrophorese aus "low-melting"-Agarose gereinigt wurde. Dieses Fragment enthält den Vektoranteil aus pUC18 und den C-terminalen Bereich von serA5. Das Plasmid pKB 1508 wurde ebenfalls mit SalI/KpnI geschnitten. Das 2,0 kb große DNS-Fragment wurde aus einem low-melting-Agarosegel eluiert. Dieses Fragment enthält das serA-Allel serA1508 mit der Insertionsmutation, jedoch fehlen die 8 C-terminalen Kodons. Die beiden Fragmente werden miteinander ligiert und damit die serA-Mutante PC1523 transformiert. Das resultierende rekombinante Plasmid wurde als pGH11 (Fig. 8) bezeichnet. Die kodierte Phosphoglyceratdehydrogenase vereinigt die Insertionsmutation von serA 1508 mit der Deletionsmutation von serA5. Die Region mit den Mutationen in der kodierten Aminosäuresequenz ist in Tabelle 1 gezeigt.

Zur Expression der mutierten serA-Allele in Produkzionsstämmen wurden sie in den Vektor pACYC 184 (ATCC37033), einen Vektor mit mittlerer Kopienzahl, unkloniert. Dazu wurden das Plasmid pGH5 und das Plasmid pGHll SalI/HindIII geschnitten und die jeweils etwa 2 kb großen, die serA-Allele serA5 und serA11 enthaltenden DNS-Fragmente aus "low-melting"-Agarose-Gelen isoliert. Die Fragmente wurden in getrennten Ansätzen mit dem Klenowfragment der DNS-PolymeraseI aus E. coli (Fa. Boehringer Mannheim GmbH) gemäß Herstellervorschrift behandelt, um die 5' überhängenden Schnittenden der Restriktionsenzyme in glatte Enden zu überführen, Dazu wurde 1 µg des jeweiligen Fragments in einem 20 µl Reaktionsansatz mit 5 U Klenowenzym, 0,5 mM dATP, dGTP, dTTP und dCTP und dem vom Hersteller empfohlenen Puffer gemischt und 15 min. bei 30°C inkubiert.

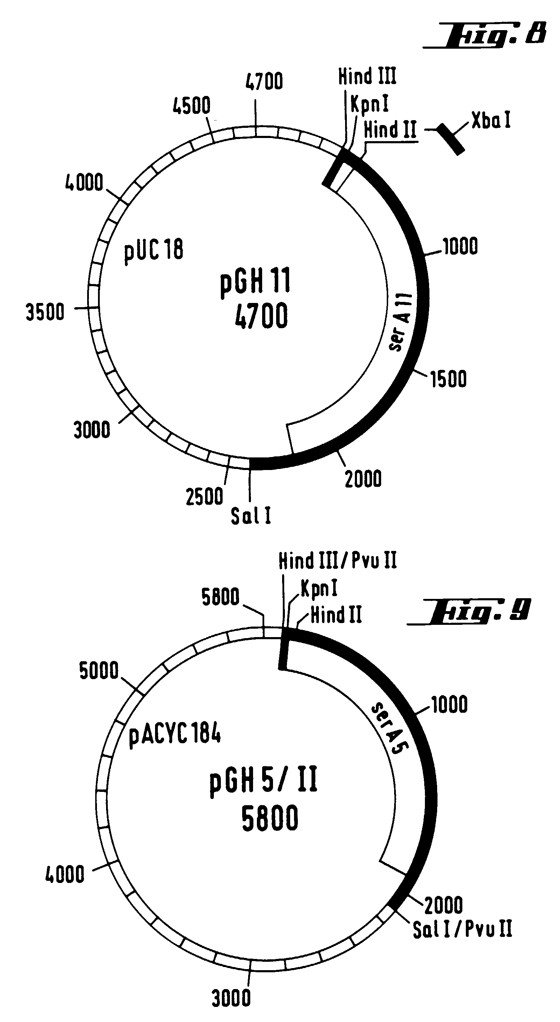

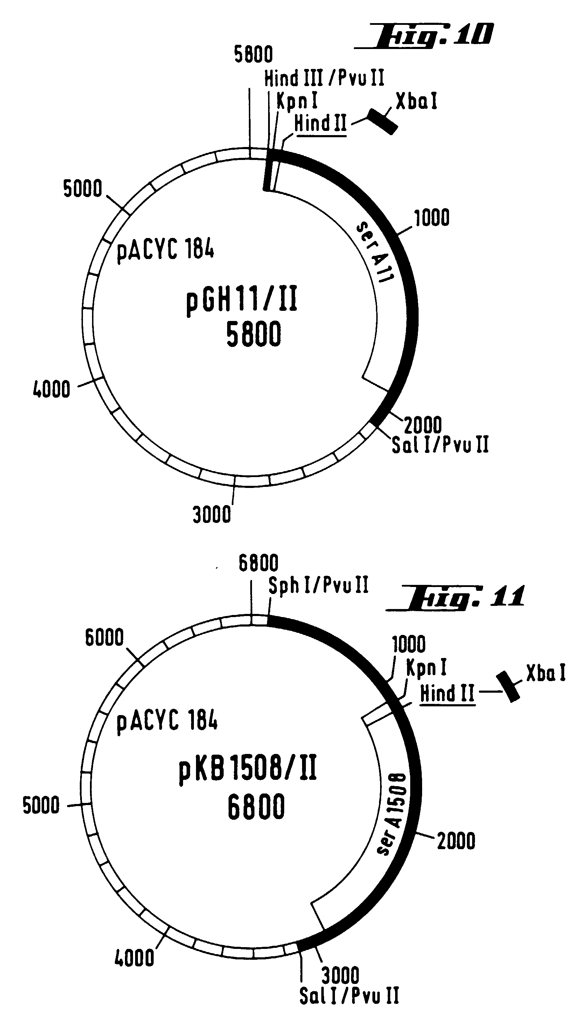

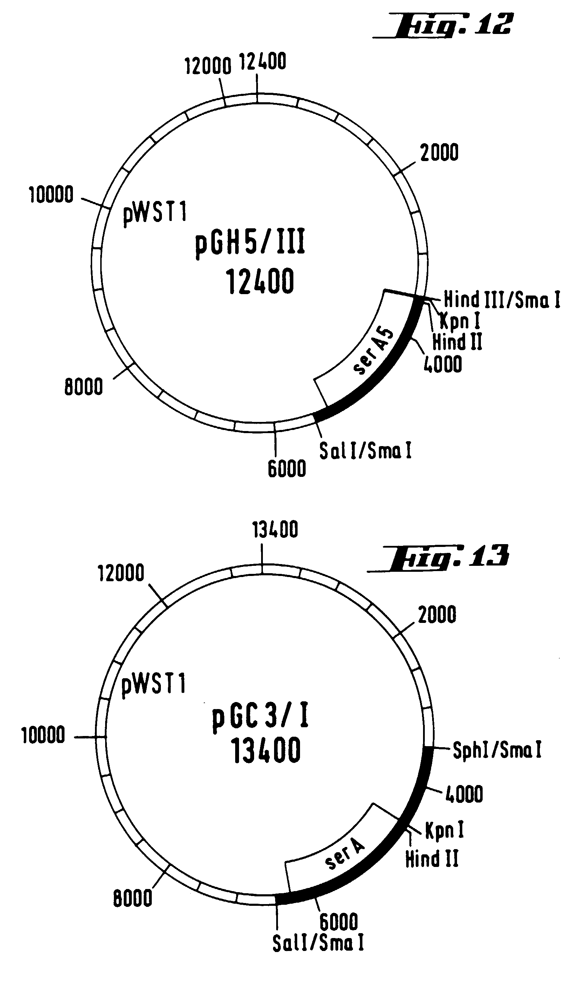

Die glattendigen DNS-Fragmente wurden jeweils mit einem PvuII geschnittenen Vektor pACYC 184 ligiert. Die Ligationsansätze wurden benützt, um die serA-Mutante PC1523 zu transformieren. Komplementierende Plasmide erhielten den Namen pGH5/II bzw. pGH11/II (Fig. 9, Fig. 10). Das Plasmid pKB1508 wurde mit SalI/SphI geschnitten. Das 3.0 kb Fragment, das das serA-Allel serA1508 enthält, wurde über Gelelektrophorese gereinigt. Das Fragment wurde, wie oben beschrieben, glattendig gemacht, mit PvuII-geschnittenem pACYC184 ligiert, und der Ligationsansatz wurde in E. coli PC1523 transformiert. Komplementierende Plasmide erhielten den Namen pKB1508/II (Fig. 11).

Die Plasmide pGH5/II (serA5), pGH11/II (serA11) und pKB 1508/II wurden benützt, um die Stämme PD103 (trpE0), KB862 (trpE5), SV164 (trpE8), SV163 (trpE6) zu transformieren.

### Beispiel 3:

### Konstruktion eines chromosomal kodierten, feedbackresistenten serA5-Allels mit Hilfe eines rekombinanten λ-Prophagen

Zur Integration in die chromosomale Lambda "attachment site" (att λ) wurde das serA5-Allel in das Plasmid pRS551 (Simons et al., 1987, Gene 53: 85-96) kloniert. Dazu wurde das ca. 2 kb große serA5-tragende HindIII/SalI-Fragment aus dem Plasmid pGH5 isoliert. Die 5'-überstehenden Enden wurden mit Hilfe des Klenow-Fragments der DNA-Polymerase I (Fa. Boehringer Mannheim GmbH) gemäß Herstellerangaben aufgefüllt und nach dem Anheften von EcoRI-Linkern (Maniatis et al., 1982, Molecular Cloning: A Laboratory Mannual, Cold Spring Harbor Laboratory, Cold Spring Harbor, N.Y.: 396-397) wurde das 2 kb Fragment in den EcoRI-gespaltenen Vektor pRS551 ligiert. Ein rekombinantes Plasmid wurde ausgewählt und mit pRS5 bezeichnet.

Durch die Herstellung eines Plattenlysats auf einem pRS5-tragenden recA⁺Stamm (z.B. YMC9 ATCC33927) mit dem λRS45-Phagen wurde in vivo durch homologe Rekombination ein heterogenes λ-Lysat erzeugt, das neben λRS45-Phagen auch rekombinante serA5-tragende λRS45-Derivate enthielt (Simons et al., 1987, Gene 53: 85-96).

Zur Selektion auf die rekombinanten λRS45-Derivate wurde der serA-Stamm PC1523 verwendet (CGSC#:5421). Dazu wurde PC1523 mit dem heterogenen λ-Lysat (siehe oben) infiziert und anschließend auf Kanamycin-haltigen (25 mg/l) LB-Platten plattiert. Die erhaltenen lysogenen, Kanamycin-resistenten Klone wurden dann auf ihre Fähigkeit getestet, auf Minimalmediumplatten ohne Serin zu wachsen. Ein Serin-prototropher Klon wurde augewählt und für die Herstellung eines homogenen serA5-λ-Lysats (durch UV-Induktion Simons et al., 1987, Gene 53: 85-96) verwendet.

Mit diesem homogenen serA5-λ-Lysat wurde der Tryptophanproduktionsstamm SV164 infiziert. Der erhaltene Stamm SV164 attλ::serA5, wurde, wie in Beispiel 4 beschrieben, fermentiert. Die jeweiligen Medien enthielten anstelle von Tetracyclin als Selektions-Agens jeweils 25 mg/l Kanamycin.

Die Ausbeuten an Tryptophan lagen bei 12,5 g/l im Vergleich zu 3,5 g/l mit dem gleichen Stamm ohne serA5.

### Beispiel 4:

### Tryptophanproduktion mit Corynebakterien

Das Plasmid pGH5 wird mit den Restriktionsenzymen SalI und HindIII (Fa. Boehringer Mannheim GmbH) geschnitten und das 2 kb große, das serA5-Gen-tragende DNS-Fragment wird aus einem "low melting"-Agarose-Gel isoliert. Das DNS-Fragment wird, wie in Beispiel 2 beschrieben, durch Einwirkung des Klenow-fragments der DNS-Polymerase I aus E. coli (Fa. Boehringer Mannheim GmbH) glattendig gemacht. Der Vektor pWST1 wird mit dem Restriktionsenzym SmaI (Fa. Boehringer) geschnitten und mit dem glattendigen DNS-Fragment ligiert. Der Vektor pWST1 ist ein E. coli/Corynebakterien-Schaukelvektor und kann sowohl in E.coli als auch in Corynebakterien replizieren. Das corynebakterielle Replikon dieses Vektors stammt aus dem Stamm Corynebakterium glutamicum ATCC 19223. Die Herstellung des Vektors pWST1 ist in US-A 4,965,197 beschrieben. Der Ligationsansatz wird benützt, um den E.coli-Stamm PC1523 zu transformieren. Komplementierende Plasmide erhalten den Namen pGH5/III (Fig. 12).

Das Plasmid pGH5/III wird benützt, um das Tryptophan-produzierende Corynebacterium glutamicum ATCC21851 zu transformieren. Die Transformation erfolgt über Elektroporation nach der bei Wolf H. et al., 1989, Appl. Microbiol. Biotechnol. 30: 283-289, ausführlich geschilderten Technik. Klone, die das rekombinante Plasmid pGH5/III tragen, werden über die Plasmid-kodierte Kanamycinresistenz auf Agarplatten mit 25 mg/l Kanamycin selektiert.

Das Plasmid pGC3 wird mit den Restriktionsenzymen SphI und SalI geschnitten. Das 3 kb DNS-Fragment, das das serA-Wildtyp-Allel trägt, wird gereinigt und nach oben beschriebener Weise in den Vektor pWST1 ligiert. Der erhaltene Vektor pGC3/I (Fig. 13) wird benützt um Corynebacterium glutamicum ATCC21851 zu transformieren.

Analog wird ein Corynebacterium glutamicum ATCC21581-Stamm hergestellt, der das serA-Allel 1455 auf einem Plasmid trägt.

In der Fermentation zeigt sich, daß der Stamm, der das serA5-Allel auf einem Plasmid trägt, die höchsten Tryptophan-Ausbeuten erreicht.

### Beispiel 5:

### Einfluß von verschiedenen plasmidkodierten serA-Allelen auf die Tryptophan-Produktion von unterschiedlichen trpE-Stämmen

Mit den verschiedenen, in Tabelle 3 zusammengefaßten Tryptophan-Produktionsstämmen wurden in einem 100 ml Erlenmeyerkolben 10 ml LB Medium (1 % Trypton, 0,5% Hefeextrakt, 0,5 % NaCl), das mit 15 mg/l Tetracyclin versetzt war, beimpft. Nach 8-9-stündiger Inkubation unter Schütteln mit 150 rpm bei 30°C wurden die jeweiligen Vorkulturen in 100 ml SM1-Medium überführt. Das SM1-Medium enthielt 5 g/l Glucose, 3 g/l KH₂PO₄, 12 g/l K₂HPO₄, 0,1 g/l (NH₄)₂SO₄, 0,3 g/l MgSO₄ x 7 H₂O, 15 mg/l CaCl₂ x 2 H₂O, 2 mg/l FeSO₄ x 7 H₂O, 1 g/l Na₃ Citrat x 2H₂O, 1 ml/l Spurenelementlösung (siehe unten), 40 mg/l L-Phenylalanin, 40 mg/l L-Tyrosin, 5 mg/l Vitamin B1 und 15 mg/l Tetracyclin. Die Spurenelenentlösung setzte sich aus 0,15 g/l Na₂MoO₄ x 2H₂O, 2,5 g/l H₃BO₃, 0,7 g/l CoCl₂ x 6H₂O, 0,25 g/l CuSO₄ x 5H₂O, 1,6 g/l MnCl₂ x 4 H₂O und 0,3 g/l ZnSO₄ x 7H₂O zusammen. Die Kulturen wurden in 1-1-Erlenmeyerkolben bei 30°C für 12-16 h mit 150 rpm geschüttelt. Nach dieser Inkubation betrug die OD₆₀₀ zwischen 2 und 4. Die weitere Fermentation wurde in Forschungsfermentern BIOSTAT^{R}M der Firma Braun-Melsungen durchgeführt. Ein Kulturgefäß mit 2 Liter Gesamtvolumen wurde benutzt.

Das Medium enthielt 17,5 g/l Glucose, 5 g/l (NH₄)₂SO₄, 0,5 g/l NaCl, 0,3 g/l MgSO₄ x 7H₂O, 15 mg/l CaCl₂ x 2 H₂O, 75 mg/l FeSO₄ x 7H₂O, 1 g/l Na₃ Citrat x 2 H₂O, 1,5 g/l KH₂PO₄, 1 ml Spurenelementlösung (siehe oben), 5 mg/l Vitamin B1 (Thiamin) , 0,75 g/l L-Phenylalanin, 0,75 g/l L-Tyrosin, 2,5 g/l Hefeextrakt (Difco), 2,5 g/l Trypton (Difco) und 20 mg/l Tetracyclin.

Die Glucosekonzentration wurde im Fermenter durch Zupumpen einer 700 g/l (w/v) Glucoselösung (autoklaviert) auf 17,5 g/l eingestellt. Vor dem Animpfen wurde dem Fermentationsmedium Tetracyclin bis zu einer Endkonzentration von 20 mg/l zugesetzt. Weiterhin wurde der pH-Wert durch Zupumpen von 25 % NH₄OH-Lösung auf 6,7 eingestellt.

100 ml Vorkultur wurden zum Animpfen in das Fermentergefäß gepumpt. Das Anfangsvolumen betrug ca. 1 l. Die Kulturen wurden zunächst mit 400 rpm gerührt und mit 1,5 vvm einer über einen Sterilfilter entkeimten Preßluft begast. Die Fermentation wurde bei einer Temperatur von 30°C durchgeführt.

Der pH-Wert wurde durch automatische Korrektur mit 25 % NH₄OH auf einem Wert von 6,7 gehalten. Die Sauerstoffsättigung in der Fermenterbrühe sollte zu keinem Zeitpunkt der Fermentation unter 20 % abfallen. Die Sauerstoffsättigung wurde bei der Fermentation über die Rührgeschwindigkeit kontrolliert.

In zwei- bis dreistündigem Abstand wurden der Glukosegehalt der Nährlösung, die optische Dichte und die Tryptophanausbeute ermittelt. Die Bestimmung des Glukosegehalts erfolgte enzymatisch mit Hilfe eines Glucoseanalysators der Firma YSI. Die Glucosekonzentration wurde durch kontinuierliches Zufüttern zwischen 5 und 20 g/l eingestellt.

Der Tryptophangehalt des Mediums nach der Fermentation wurde mittels HPLC bestimmt. Aufgetrennt wurde das Medium auf einer Nucleosil 100-7/C8 (250/4 mm; Macherey-Nagel). Die Säule wurde isokratisch mit einer Flußrate von 2 ml/min betrieben. Als Fließmittel wurde Wasser/Acetonitril (83/17), welchem pro Liter 0,1 ml H₃PO₄ (85%) zugesetzt war, verwendet. Detektiert wurde entweder mit einem Diodenarraydetektor oder bei einer Festwellenlänge von 215 bzw. 275 nm. Nach 44-50 h wurde die Fermentation abgebrochen. Die produzierten Tryptophanmengen in g/l dieser Fermentationen nach 48 h sind in Tabelle 3 zusammengefaßt.

**Tabelle 3:**

| | | | | | |
|---|---|---|---|---|---|
| **Tryptophanausbeuten mit verschiedenen serA/trpE-Kombinationen** | | | | | |

| | serAWT | serA5 | serA1508 | serA11 | serA1455 |
|---|---|---|---|---|---|
| trpE0 | 15,7 | 20,2 | n. b. | n. b. | 6,7 |
| trpE5 | 12,5 | 18,9 | 15,0 | 20,0 | 7,5 |
| trpE8 | 11,6 | 24,1 | 13,8 | 24,0 | 4,0 |
| trpE6 | 7,5 | 18,0 | n. b. | 11,5 | 3,9 |
| n. b. nicht bestimmt | | | | | |

## Patentansprüche

1. Stämme von Mikroorganismen für die Produktion von Tryptophan, dadurch gekennzeichnet, daß sie einen deregulierten Tryptophanstoffwechsel und einen durch ein feedbackresistentes serA-Allel deregulierten Serinstoffwechsel besitzen, wobei das serA-Allel einen Kᵢ-Wert für Serin zwischen 0,1 mM und 50 mM hat.

2. Stämme nach Anspruch 1, dadurch gekennzeichnet, daß das trpE-Allel einen Kᵢ-Wert für Tryptophan zwischen 0,1 mM und 20 mM hat.

3. Stämme nach Anspruch 1 oder 2, dadurch gekennzeichnet, daß das serA-Allel in das Chromosom integriert ist.

4. Stämme nach einem oder mehreren der Ansprüche 1 bis 3, dadurch gekennzeichnet, daß die Mikroorganismen Bakterien sind.

5. Stämme nach Anspruch 4, dadurch gekennzeichnet, daß die Bakterien zur Spezies E. coli gehören.

6. Stämme nach Anspruch 4, dadurch gekennzeichnet, daß die Bakterien zur Species Corynebakterium gehören.

7. Verfahren zur Herstellung von Stämmen gemäß Anspruch 1, dadurch gekennzeichnet, daß in einen Mikroorganismenstamm mit dereguliertem Tryptophanstoffwechsel ein feedbackresistentes serA-Allel eingebracht wird.

8. Verfahren gemäß Anspruch 7, dadurch gekennzeichnet, daß das serA-Allel ins Chromosom des Mikroorganismus mit dereguliertem Tryptophanstoffwechsel eingebracht wird.

9. Verwendung von Stämmen nach einem oder mehreren der Ansprüche 1 bis 6 zur Produktion von Tryptophan.

## Claims

1. Strains of microorganisms for the production of tryptophan, characterised in that they have a deregulated tryptophan metabolism and a serine metabolism which is deregulated by a feedback-resistant serA allele, where the serA allele has a Kᵢ value for serine between 0.1 mM and 50 mM

2. Strains according to Claim 1, characterised in that the trpE allele has a Kᵢ value for tryptophan between 0.1 mM and 20 mM.

3. Strains according to Claim 1 or 2, characterised in that the serA allele is integrated into the chromosome.

4. Strains according to one or more of Claims 1 to 3, characterised in that the microorganisms are bacteria.

5. Strains according to Claim 4, characterised in that the bacteria belong to the species E. coli.

6. Strains according to Claim 4, characterised in that the bacteria belong to the species [sic] corynebacterium.

7. Process for the preparation of strains according to Claim 1, characterised in that a feedback-resistant serA allele is introduced into a microorganism strain with deregulated tryptophan metabolism.

8. Process according to Claim 7, characterised in that the serA allele is introduced into the chromosome of the microorganism with deregulated tryptophan metabolism.

9. Use of strains according to one or more of Claims 1 to 6 for the production of tryptophan.

## Revendications

1. Souches de micro-organismes producteurs de tryptophane, caractérisées en ce qu'elles possèdent un métabolisme du tryptophane dérégulé et un métabolisme de la sérine dérégulé par un allèle SerA résistant à la rétroaction, où l'allèle SerA a une valeur Kᵢ pour la sérine comprise entre 0,1 mM et 50 mM.

2. Souches suivant la revendication 1, caractérisées en ce que l'allèle trpE a une valeur Kᵢ pour le tryptophane comprise entre 0,1 mM et 20 mM.

3. Souches suivant la revendication 1 ou 2, caractérisées en ce que l'allèle SerA est intégré dans le chromosome.

4. Souches suivant l'une ou plusieurs des revendications 1 à 3, caractérisées en ce que les micro-organismes sont des bactéries.

5. Souches suivant la revendication 4, caractérisées en ce que les bactéries appartiennent à l'espèce E. coli.

6. Souches suivant la revendication 4, caractérisées en ce que les bactéries appartiennent à l'espèce Corynebacterium.

7. Procédé de préparation de souches suivant la revendication 1, caractérisé en ce que l'on introduit un allèle SerA résistant à la rétroaction, dans une souche de micro-organisme avec métabolisme du tryptophane dérégulé.

8. Procédé suivant la revendication 7, caractérisé en ce que l'allèle SerA est introduit dans le chromosome du micro-organisme avec métabolisme du tryptophane dérégulé.

9. Utilisation des souches suivant l'une ou plusieurs des revendications 1 à 6, pour la production de tryptophane.
